# EUROPEAN PATENT SPECIFICATION

(11) **EP 0 555 629 B1**
(45) Date of publication and mention of the grant of the patent: **09.08.1995**
(21) Application number: 93100111.9
(22) Date of filing: 07.01.1993
(51) Int. Cl.: A61F 2/36

(54) **A femoral member of a hip joint endoprosthesis**
Oberschenkelteil einer Hüftgelenkendoprothese
Partie fémorale d'une endoprothèse pour l'articulation de la hanche

(30) Priority: 11.02.1992 DE 9201646 U
(43) Date of publication of application: 18.08.1993
(73) Proprietor: Howmedica GmbH, D-24232 Schönkirchen (DE)
(72) Inventor: Vécsei, Vilmos, A-1090 Vienna (AT); Harder, Hans Erich, D-2316 Probsteierhagen (DE); Obersteiner, Karl, CH-9434 AU (CH)
(74) Representative: Patentanwälte Hauck, Graalfs, Wehnert, Döring, Siemons

(56) References cited:
- EP-A- 0 012 146
- WO-A-86/06954
- DE-U- 9 006 893
- US-A- 3 466 670
- US-A- 4 865 608

## Description

The invention relates to a femoral member of a hip joint endoprosthesis according to the preamble of claim 1.

Conventional endoprostheses for the hip joint mostly have a solid shaft which is straight or bent and which is anchored in the femur canal with or without bone cement. Femoral members implanted using bone cement are difficult to surgically remove when they become loosened, for example. Shafts implanted free of cement generate a frictional connection between the shaft and the bone which sometimes results in substantial tensions in the bone canal. EP-A 0 309 662 or EP-A-0 243 585 disclose prosthesis shafts having a box-like profile, where-in the cavity is accessible from outside through dorsal and frontal openings. The cavity may be filled with endogenic spongiosa to facilitate the taking of the prosthesis in the femur by a genuine bony connection to obtain a safe anchoring which is maintained for a long time. A box-like shaft profile undoubtedly has an increased elasticity so that the deformability of the materials approaches each other in the boundary region between the prosthesis shaft and the bone. On the other hand, there is the danger that locally high tension peaks develop which are detrimental to the stability of the shaft. This, however, can be counter-acted by molding the shaft as disclosed in EP-A 0 309 662. US-A-3 466 670 discloses a bent, relatively slim hip pros-thesis having circular openings in the proximal shaft portion extending from ventral to dorsal, wherein the diameter of the openings decreases towards distal. The openings have the purpose to reduce the weight of the shaft.

From EP-A-12146 a femoral member is known which has a shank approximately rectangular in cross section. Substantially the complete length of the shank is provided with recesses around the shank to provide stabilizing ribs. Only immediately adjacent to the proximal end of the shank two windows are provided adapted to accommodate spongy material. These windows are elongated with the longer axis extending approximately perpendicular to the medial side of the shank.

WO-A-8606956 discloses a femoral member for a hip joint endoprosthesis as specified in the pre-characterizing portion of claim 1, which comprises a shank having a medial and a lateral side which both are arcuately rounded in cross section. The complete shank is provided with holes connecting the dorsal side with the ventral side of the shank. The holes adjacent to the proximal portion of the shank are elongated, with the longer axis extending tranverse to the axis of the shank. The distal holes of the known femoral member are essentially closed by suitable plugs to prevent the bone from going into the holes.

It is an object of the invention to provide a femoral member for a hip joint endoprosthesis to be implanted free of cement which affords the best possible connection between the femoral member and the bone by providing a sufficient stability of the prosthesis and a simple surgical operation.

This object is attained by the features of claim 1.

The femoral member according to the invention has a distal portion which is approximately straightly shaped, wherein according to an embodiment of the invention the cross section of the distal portion is approximately circular or oval. The proximal portion of the shaft has four to six, preferably five, elongated holes with the longer axis thereof extending under an angle to the axis of the distal shaft portion between 50 and 65°, preferably about 60°. The elongated holes are relatively long and at least the three elongated holes adjacent to the proximal end of the shaft have a length which is equal to or longer than twice of the width thereof. The holes are freely open for the accommodation of spongiosa. The webs between the elongated holes are relatively small, preferably smaller than the width of the holes.

The arrangement and angular position of the holes result in an optimum pressure distribution in the proximal shaft portion. Furthermore, relatively large cavities are provided to be filled up with spongiosa for metaphysar stabilization.

The shaft of the femoral member according to the invention is relatively slim and facilitates a simple surgical operation and a standardization for a variety of applications. Laterally and medially the shaft comes into contact with the wall of the femur which is widened by a drilling operation. According to an embodiment of the invention, the distal shaft portion has a central bore, thus relieving the medullary canal. In a further embodiment of the invention, the central bore may extend through the complete shaft and still further may end in a proximal projection which extends approximately perpendicular to the bore. The projection can be used as impact face in driving the shaft into the medullary canal. By means of the central bore, a lance may be used as inserting means. The lance is anchored in the bone before the shaft is driven in and thus affords a guide while the shaft is being driven in. Proximally, the bore preferably has an internal thread which is used for mounting an impact device for example, or for mounting an extracting tool.

According to a further embodiment of the invention, the shaft is covered with hydroxylapatit in the area of the holes to afford a more effective taking. The shaft may be made of a suitable body-compatible material, preferably by molding. Preferably a chromium cobalt or titan alloy is used.

An embodiment of the invention is now described with reference to the drawings.
- Fig. 1: shows a ventral and dorsal side of a femoral member of a hip joint endoprosthesis according to the invention,
- Fig. 2: shows the lateral side of the femoral member of Fig. 1.
- Fig. 3: shows a similar view of the proximal shaft portion, partly in section,
- Fig. 4: shows a section of the illustration of Fig. 1 along line 4-4,
- Fig. 5: shows a section of the illustration of Fig. 1 along line 5-5,
- Fig. 6: shows a section of the illustration of Fig. 1 along line 6-6,
- Fig. 7: shows a section of the illustration of Fig. 6 along line 7-7,
- Fig. 8: shows a section of the illustration of Fig. 1 along line 8-8.

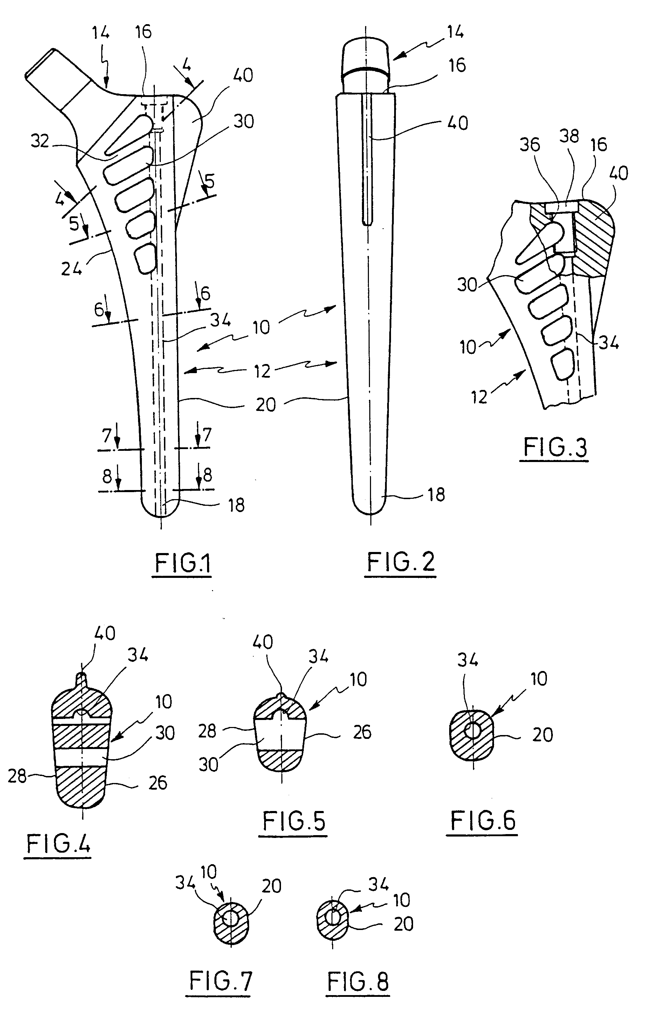

The femoral member 10 shown in Figs. 1 to 3 has a shaft 12 and a collar 14. The collar is not described in more detail since it is conventional. As shown, the shaft 12 merges into the tapered neck portion without forming a collar. Therebetween, a projection 16 is formed laterally with respect to the neck portion, which projection functions as an impact surface for driving home the femoral member. The projection 16 or, respectively the impact surface, extend approximately vertical to the axis 18 extending through the shaft 10.

As shown, the lower third (distal portion) shown at 20 is shaped approximately straight, having an approximately circular or approximately oval cross-section as shown in Figs. 6 to 8. As shown in Fig. 1, the complete lateral side which cross-section is arcuately bent in the remaining portion, extends straight up to the projection 16. The proximal shaft portion 24 is, however, medially slightly arcuately bent as shown in Figs. 1 and 3. Frontal side 26 and dorsal side 28 are flat (Figs. 4 and 5) and include medially a very small angle.

The proximal shaft portion 24 has five holes 30 extending from the ventral to the dorsal side. The four upper holes 30 of Fig. 1 are shaped as elongated holes, wherein the three central holes 30 have an approximately rectangular cross-section. The uppermost hole 30 is shaped approximately triangular and increases in width towards lateral. The longer axes of the elongated holes 30 approximately extend under an angle of 60° to the axis 18. The lowermost hole 30 is an elongate hole of which the longer axis is parallel to the axis 18. As shown, the holes 30 approximately end in an equal distance from the medial and lateral side. The length is at least half as large as the width of the shaft portion 24 in this region. The webs 32 between the holes 30 are relatively small. In the illustration they are smaller than half the width of the holes 30. The centers of the holes 30 are located on a bent curve which smoothely merges into the axis 18 in the distal portion 20. The length of the three upper elongated holes is larger than half the width of the holes 30. A large receiving space for spongiosa results, but the stability of the shaft is not worsened. The angular location of the openings 30 affords a fair pressure distribution in the shaft 10. A bore 34 through the shaft 12 extends coaxially with respect to the distal portion 10, which bore 34 proximally ends in the impact surface 16. As shown in Fig. 3 the bore 34 is broadened at 36 and bears an internal thread for receiving a tool. The bore portion 36 can be closed by a screw after driving home the femoral member 10, wherein the head of a screw may be mounted flush in the broadened portion 38. The bore 18 affords driving home the femoral member 10 by means of a guide lance which has been driven in the femur before. Laterally the proximal portion 24 has a web 40 to secure the implanted femoral member 10 against rotation.

The femoral member 10 is made of a suitable body-compatible alloy, for example a chromium cobalt alloy or, preferably, a titan alloy be molding. The member is made in a set of stepped lengths and diameters.

## Claims

1. A femoral member of a hip joint endoprosthesis, comprising a shaft (20) having a medial and a lateral side which both are arcuately rounded in cross section and having a proximal portion which is provided with elongated holes (30), connecting the dorsal side with the ventral side, wherein the longer axis of the holes (30) which extends substantially transverse to the axis of the shank (20) decreases from the proximal to the distal end of the shank (20), the distal portion of the shank (20) being formed approximately straight, characterized in that only the proximal shank portion is provided with four to six approximately parallel elongated holes with the longer axis thereof extending under an angle to the axis (18) of the distal shaft portion between 50 and 65°, preferably about 60°, and approximately perpendicular to the medial side of the shaft which is proximally slightly curved, in that further the longer axis at least of the first elongated holes (30) adjacent to the proximal end of the shaft is equal to or larger than twice the width thereof, in that the holes (30) are freely open for the accommodation of spongiosa, and in that the lateral side of the shaft (20) is substantially straight in distal-proximal direction.

2. The femoral member of claim 1, characterized in that the width of the webs (32) between the openings (30) is smaller than the width of the openings, preferably at most half as broad as the openings (30).

3. The femoral member of claim 1 or 2, characterized in that the distal shaft portion (20) has an approximately circular or oval cross section.

4. The femoral member of one of claims 1 to 3, characterized in that the dorsal and the ventral side (26, 28) of the proximal shaft portion (24) are flat and include a small angle towards medial.

5. The femoral member of one of claims 1 to 4, characterized in that a coaxial bore (34) preferably extending through the complete shaft (12) is formed in the distal shaft portion (20).

6. The femoral member of claim 5, characterized in that the bore (34) ends proximally in a projection (16) of the shaft (12) which extends approximately perpendicular to the bore (34).

7. The femoral member of one of claims 1 to 6, characterized in that the shaft (12) is covered with hydroxylapatit in the region of the elongated holes (30).

8. The femoral member of one of claims 1 to 7, characterized in that the shaft (12) is made of a chromium cobalt or titan alloy by molding.

## Patentansprüche

1. Oberschenkelteil einer Hüftgelenkendoprothese, mit einem Schaft (20), der eine mediale und laterale Seite aufweist, die beide im Querschnitt bogenförmig gerundet sind, und einen proximalen Abschnitt, der die dorsale Seite mit der ventralen verbindende Langlöcher (30) aufweist, wobei die längere Achse der Löcher (30), die sich im wesentlichen quer zu der Achse des Schafts (20) erstreckt, von dem proximalen zu dem distalen Ende des Schafts (20) hin abnimmt, der distale Abschnitt des Schafts (20) annähernd gerade geformt ist, dadurch gekennzeichnet, daß nur der proximale Schaftabschnitt mit vier bis sechs annähernd parallelen Langlöchern versehen ist, deren längere Achse sich unter einem Winkel zu der Achse (18) des distalen Schaftabschnitts zwischen 50 und 65°, vorzugsweise etwa 60°, erstreckt und annähernd senkrecht zur medialen Seite des Schafts, die proximal schwach gekrümmt ist, dadurch, daß ferner die längere Achse zumindest der dem proximalen Ende des Schafts benachbarten ersten Langlöcher (30) gleich oder größer ist als deren doppelte Breite, dadurch, daß die Löcher (30) frei zugänglich für die Aufnahme von Spongiosa sind, und dadurch, daß die laterale Seite des Schafts (20) im wesentlichen gerade in distaler-proximaler Richtung verläuft.

2. Oberschenkelteil nach Anspruch 1, dadurch gekennzeichnet, daß die Breite der Stege (32) zwischen den Öffnungen (30) kleiner ist als die Breite der Öffnungen, vorzugsweise höchstens halb so breit wie die Öffnungen (30).

3. Oberschenkelteil nach Anspruch 1 oder 2, dadurch gekennzeichnet, daß der distale Schaftabschnitt (20) einen annähernd kreisförmigen oder ovalen Querschnitt aufweist.

4. Oberschenkelteil nach einem der Ansprüche 1 bis 3, dadurch gekennzeichnet, daß die dorsale und die ventrale Seite (26, 28) des proximalen Schaftabschnitts (24) flach sind und nach medial einen kleinen Winkel einschließen.

5. Oberschenkelteil nach einem der Ansprüche 1 bis 4, dadurch gekennzeichnet, daß im distalen Schaftabschnitt (20) eine koaxiale, vorzugsweise durch den gesamten Schaft (12) hindurchgehende Bohrung (34) geformt ist.

6. Oberschenkelteil nach Anspruch 5, dadurch gekennzeichnet, daß die Bohrung (34) proximal in einem Absatz (16) des Schafts (12) endet, der sich annähernd senkrecht zur Bohrung (34) erstreckt.

7. Oberschenkelteil nach einem der Ansprüche 1 bis 6, dadurch gekennzeichnet, daß der Schaft (12) in dem Bereich der Langlöcher (30) mit Hydroxylapatit beschichtet ist.

8. Oberschenkelteil nach einem der Ansprüche 1 bis 7, dadurch gekennzeichnet, daß der Schaft (12) aus einer Chromkobalt- oder einer Titanlegierung gegossen ist.

## Revendications

1. Elément fémoral pour une endoprothèse de l'articulation de la hanche, comportant une tige (20) ayant un côté interne et un côté latéral qui ont tous deux une section transversale arrondie en arc et ayant une partie proximale qui est pourvue de trous allongés (30), reliant le côté dorsal au côté ventral, dans lequel le grand axe des trous (30), qui s'étend sensiblement transversalement à l'axe de la tige (20), diminue de l'extrémité proximale vers l'extrémité distale de la tige (20), la partie distale de la tige (20) étant formée de façon à être approximativement droite, caractérisé en ce que seule la partie proximale de la tige est pourvue de quatre à six trous allongés approximativement parallèles dont le grand axe s'étend en formant, avec l'axe (18) de la partie distale de la tige, un angle compris entre 50 et 65°, avantageusement d'environ 60°, et est approximativement perpendiculaire au côté interne de la tige qui est légèrement incurvé dans le sens proximal, en ce qu'en outre le grand axe au moins des premiers trous allongés (30) adjacents à l'extrémité proximale de la tige est égal ou supérieur au double de la largeur de ces trous, en ce que les trous (30) sont à ouverture libre pour recevoir le tissu spongieux, et en ce que le côté latéral de la tige (20) est sensiblement droit dans le sens distal-proximal.

2. Elément fémoral selon la revendication 1, caractérisé en ce que la largeur des voiles (32) entre les ouvertures (30) est inférieure à la largeur des ouvertures, avantageusement au plus égale à la moitié de la largeur des ouvertures (30).

3. Elément fémoral selon la revendication 1 ou 2, caractérisé en ce que la partie distale (20) de la tige présente une section transversale approximativement circulaire ou ovale.

4. Elément fémoral selon l'une des revendications 1 à 3, caractérisé en ce que les côtés dorsal et ventral (26, 28) de la partie proximale (24) de la tige sont plats et forment un petit angle vers le côté interne.

5. Elément fémoral selon l'une des revendications 1 à 4, caractérisé en ce qu'un alésage coaxial (34) s'étendant avantageusement sur toute la tige (12) est formé dans la partie distale (20) de la tige.

6. Elément fémoral selon la revendication 5, caractérisé en ce que l'alésage (34) se termine du côté proximal dans une saillie (16) de la tige (12), laquelle saillie s'étend à peu près perpendiculairement à l'alésage (34).

7. Elément fémoral selon l'une des revendications 1 à 6, caractérisé en ce que la tige (12) est recouverte d'hydroxylapatite dans la zone des trous allongés (30).

8. Elément fémoral selon l'une des revendications 1 à 7, caractérisé en ce que la tige (12) est réalisée par moulage en alliage de chrome, de cobalt ou de titane.
